# EUROPÄISCHE PATENTSCHRIFT

(11) **EP 1 265 837 B1**
(45) Veröffentlichungstag und Bekanntmachung des Hinweises auf die Patenterteilung: **09.06.2004**
(21) Anmeldenummer: 00977582.6
(22) Anmeldetag: 27.11.2000
(51) Int. Cl.: C07C 51/60, C07C 63/10

(54) **VERFAHREN ZUR HERSTELLUNG VON O-CHLORMETHYLBENZOESÄURECHLORIDEN**
METHOD FOR PRODUCING O-CHLOROMETHYL BENZOIC ACID CHLORIDES
PROCEDE DE FABRICATION DE CHLORURES D'ACIDE O-CHLOROMETHYLBENZOIQUE

(30) Priorität: 07.12.1999 DE 19958757; 19.02.2000 DE 10007694
(43) Veröffentlichungstag der Anmeldung: 18.12.2002
(73) Patentinhaber: BASF AKTIENGESELLSCHAFT, 67056 Ludwigshafen (DE)
(72) Erfinder: GÖTZ, Roland, 68809 Neulussheim (DE); GÖTZ, Norbert, 67547 Worms (DE); KEIL, Michael, 67251 Freinsheim (DE); WOLF, Bernd, 67136 Fussgönheim (DE); STEINMETZ, Adrian, 68165 Mannheim (DE); STAMM, Armin, 55268 Nieder-Olm (DE); HENKELMANN, Jochem, 68165 Mannheim (DE)
(74) Vertreter: Fitzner, Uwe, Dr.
(86) Internationale Anmeldenummer: PCT/EP2000/011817
(87) Internationale Veröffentlichungsnummer: WO 2001/042185

(56) Entgegenhaltungen:
- EP-A- 0 676 389
- WO-A-97/12854
- WO-A-99/16743

## Beschreibung

Die vorliegende Erfindung betrifft ein Verfahren zur Herstellung von o-Chlormethylbenzoesäurechloriden der Formel I, 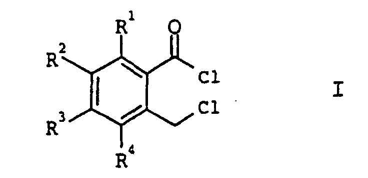 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 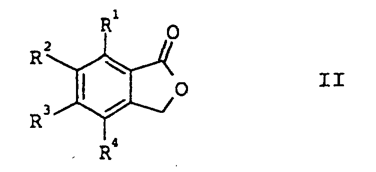 in der R¹ bis R⁴ die vorgenannte Bedeutung besitzen, mit Thionylchlorid.

o-Chlormethyl-substituierte Benzoylchloride sind wichtige Zwischenprodukte zur Herstellung von beispielsweise pestiziden Wirkstoffen wie sie in den Patentschriften EP-A 460 575, EP-A 463 488, WO-A 95/18789, WO-A 95/21154 und WO-A 97/15552 beschrieben sind.

o-Chlormethyl-substituierte Benzoylchloride können zum Beispiel durch Umsetzung von benzokondensierten Lactonen mit Thionylchlorid oder Phosgen hergestellt werden. Bei der Verwendung von Thionylchlorid vereinfacht sich die Apparatetechnik und der sicherheitstechnische Aufwand verringert sich.

In EP-A 676 389 wird die Herstellung von o-Chlormethylbenzoesäurechloriden aus benzokondensierten Lactonen mit Thionylchlorid in Gegenwart einer Stickstoffverbindung beschrieben. Um einen befriedigenden Umsatz zu erzielen, sind Reaktionstemperaturen von 160 bis 170°C erforderlich, bei denen sich Thionylchlorid bereits teilweise zersetzt und infolgedessen störende Nebenprodukte gebildet werden. Weiterhin ist die Zugabe von gasförmiger Salzsäure erforderlich. Schließlich liegen die Ausbeuten zum Teil deutlich unter 90%.

WO 97/12854 beschreibt ein Verfahren zur Herstellung von o-Chlormethylbenzoesäurechloriden durch Phosgenierung von benzokondensierten Lactonen in Gegenwart eines Triarylphosphinoxid-Katalysators bei 170°C. Zwar ist Phosgen unter diesen Bedingungen im Gegensatz zu Thionylchlorid thermisch stabil, jedoch wird die Handhabung von Phosgen und dessen Holdup im Kühler bei den hohen Temperaturen durch einen erhöhten sicherheitstechnischen Aufwand erschwert. Des weiteren wird das Reaktionsprodukt unter diesen Bedingungen thermisch stark belastet, was zu seiner teilweisen Zersetzung führen kann.

In der WO-A 99/16743 wird die Umsetzung mit Thionylchlorid in Gegenwart eines quartären Ammoniumsalzes und einer Lewis-Säure bei 90 und 100°C durchgeführt. Quartäre Ammoniumsalze sind jedoch unter Umweltaspekten problematisch und haben folgende verfahrenstechnische Nachteile: durch Sublimation kann es zur Verstopfung von Anlagenteilen kommen. Weiterhin sind die Salze hygroskopisch, was zum Wassereintrag und einem Mehrverbrauch am Chlorierungsmittel führen kann. Schließlich stören die Ammoniumsalze bei der destillativen Aufreinigung der o-Chlormethylbenzoesäurechloride.

Aufgabe der vorliegenden Erfindung war es daher ein wirtschaftliches und prozeßfähiges Verfahren zur Herstellung von o-Chlormethylbenzoesäurechloriden zu finden, das die oben aufgeführten Nachteile nicht aufweist und dennoch hohe Ausbeuten liefert.

Demgemäß wurde das eingangs erwähnte Verfahren gefunden, das dadurch gekennzeichnet ist, daß man die Umsetzung in Gegenwart katalytischer Mengen einer Lewis-Säure und katalytischer Mengen eines Phosphinderivats der Formel III 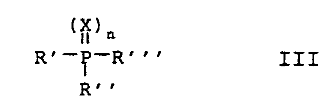 durchführt, in der R' bis R''' gleich oder verschieden sein können und C₁-C₁₀-Alkyl oder unsubstituiertes oder durch C₁-C₄-Alkyl substituiertes Phenyl bedeuten und der Index n für 0 oder 1 steht. X steht für Sauerstoff oder zwei einfach gebundene Chloratome.

Zum Einsatz gelangen benzokondensierte Lactone (Phthalide) der allgemeinen Formel II 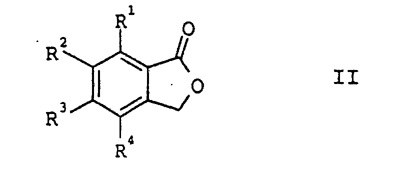 in der R¹ bis R⁴ gleich oder verschieden sein können und für Wasserstoff (H), C₁-C₄-Alkyl, Halogen (Fluor, Chlor, Brom oder Iod) oder Trifluormethyl stehen. Bevorzugt wird unsubstituiertes Phthalid eingesetzt

Als Katalysator wird zum einen ein Phosphin oder Phosphinoxid der Formel III 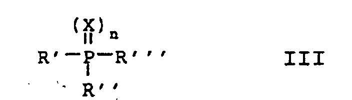 eingesetzt, in der R' bis R''' gleich oder verschieden sein können und C₁-C₁₀-Alkyl oder unsubstituiertes oder durch C₁-C₄-Alkyl substituiertes Phenyl bedeuten. Der Index n steht für 0 oder 1 und X steht für Sauerstoff oder zwei einfach gebundene Chloratome Bevorzugt ist unsubstituiertes Triphenylphosphinoxid.

Der Einsatz von bei Raumtemperatur flüssigen Trialkylphosphinoxiden hat insbesondere verfahrenstechnische Vorteile (Vermeidung des Feststoffhandlings, einfacher Austrag des Destillationsrückstandes bei der Aufreinigung) und ist daher ebenfalls bevorzugt. Hier kommen beispielsweise die unter dem Handelsnamen Cyanex® erhältlichen Tri(C₆-C₈-alkyl)phosphinoxide (s. z.B. Cyanex® 923 der Fa. Cyanamid) in Betracht. Insbesondere haben sich die flüssigen Trialkylphosphinoxide in der Kombination mit Lewis-Säuren wie Borsäure, Borsäuretri (C₁-C₄-alkyl) ester und Bortrifluorid-Addukten bewährt.

Das Phosphinderivat wird in der Regel in Mengen von 0,1 bis 20 mol-% bezogen auf die eingesetzte Menge an benzokondensiertem Lacton, und bevorzugt in Mengen von 0,5 bis 10 mol-% zugesetzt.

Als Lewis-Säure eignen sich insbesondere Borverbindungen wie BF3, BCl₃ (bzw. deren Komplexe mit Sauerstoff-, Schwefel- oder Stickstoffverbindungen), Boronsäuren - z.B. Arylboronsäuren (insbesondere Phenylboronsäure), deren C₁-C₄-Alkylester sowie C₁-C₆-Alkylboronsäuren und deren C₁-C₄-Alkylester -, cyclische Borsäurester (insbesondere Tris(C₁-C₄-Alkoxy)boroxin), Borsäuretri(C₁-C₄-alkyl) ester, Borsäureanhyrid, Borat (insbesondere Natriumborat/Borax) und die Borsäure (H₃BO₃) selbst. Desweiteren kommen heterogene, Lewis-saure Aluminiumsilikate vom Zeolith-Typ in Frage.

Bevorzugt sind BF₃ und deren Komplexe mit Ether (insbesondere Diethylether), Wasser (Dihydrat), Alkohol (insbesondere Methanol), Sulfid (insbesondere Dimethylsulfid) und Amin (insbesondere Ethylamin). Insbesondere geeignet sind BF₃-Etherat und BF₃-Dihydrat.

Besonders bevorzugt werden Borsäure, Borsäuretri (C₁-C₄-alkyl)-ester oder cyclische Borsäureester als Lewis-Säuren eingesetzt. Als cyclische Borsäureester kommen beispielsweise Trimethoxyboroxin und Triethanolaminborat in Frage. Derartige Verfahren liefern hervorragende Ausbeuten und haben den Vorteil, daß die Reaktionsgemische frei von Fluoridionen sind. Damit vereinfacht sich im Vergleich zur analogen Reaktion mit BF₃-Derivaten als Lewis-Säure die gesamte Apparatetechnik.

Die Lewis-Säure wird in Mengen von 0,1 bis 20 mol-% bezogen auf die eingesetzte Menge an benzokondensiertem Lacton, bevorzugt in Mengen von 0,5 bis 5 mol-%, zugegeben.

Weiterhin kann es von Vorteil sein heterogene, Lewis-saure Katalysatoren wie z. B. Zeolithe vom Faujasit-Typ, bei denen austauschbare Kationen teilweise oder vollständig durch Protonen ersetzt sind, einzusetzen. Eine heterogen katalysierte Reaktion hat den Vorteil, daß sie im Festbett durchgeführt werden kann. Der heterogene Katalysator wird in Mengen von 0,01 bis 10 Gewichts-% und bevorzugt in Mengen von 0,1 bis 1 Gewichts-% bezogen auf die eingesetzte Menge an benzokondensiertem Lacton eingesetzt.

Im Bezug auf das Phthalid II werden im allgemeinen 1 bis 1,5 Äquivalente Thionylchlorid eingesetzt.

Das Thionylchlorid kann mit den anderen Reaktionspartnern vorgelegt werden (batch-Fahrweise) oder im Verlauf der Reaktion vorzugsweise innerhalb von 1 bis 8 Stunden zudosiert werden (semibatch-Fahrweise). Weiterhin ist es möglich die Reaktion kontinuierlich durchzuführen.

Optional kann zur Beschleunigung der Ringöffnung gasförmiger Chlorwasserstoff eingeleitet werden. Bevorzugt wird jedoch auf die Einleitung von Chlorwasserstoff während der Synthese verzichtet.

Die Reaktionstemperatur beträgt im Falle der Borhalogenide im allgemeinen 80 bis 140°C und vorzugsweise 90 bis 110°C. Werden Borsäure oder Borsäuretri(C₁-C₄-alkyl)ester eingesetzt, beträgt die Reaktionstemperatur in der Regel 100 bis 180°C und vorzugsweise 110 bis 140°C.

Das Verfahren wird vorzugsweise ohne Lösungsmittel durchgeführt. Es ist jedoch möglich ein gegen Thionylchlorid inertes Lösungsmittel zuzusetzen. Inerte Lösungsmittel sind z. B. aromatische Kohlenwasserstoffe wie Toluol, o-, m- oder p-Xylol oder deren Gemische, chlorierte aromatische Kohlenwasserstoffe wie Chlorbenzol oder Dichlorbenzolen oder cyclische Carbonate wie Ethylen- oder Propylencarbonat. Weiterhin ist es möglich, Thionylchlorid selbst als Lösungsmittel einzusetzen, das am Ende der Reaktion destillativ abgetrennt und erneut in den Prozeß eingeschleust werden kann.

Die Reaktion wird im allgemeinen bei Normaldruck oder bei 1 bis 10 bar Druck durchgeführt.

Die nachfolgenden Beispiele sollen das Verfahren weiter erläutern.

### Verfahrensbeispiele

### Allgemeine Vorschrift zur Herstellung von o-Chlormethylbenzoylchlorid

In einer Rührapparatur bestehend aus einem 1,6 l Doppelmantelreaktor mit aufgesetzter Intensivkühlungskaskade wurden jeweils x mol Phthalid zusammen mit dem jeweiligen Katalysatorsystem vorgelegt. 1,3 Äqivalente Thionylchlorid bezogen aufs Phthalid wurden entweder mit den anderen Komponenten vorgelegt oder während des Zeitraums von 1 bis 8 Stunden zugetropft. Danach ließ man 1 bis 15 Stunden bei Reaktionstemperatur nachrühren. Der Wertgehalt der Rohmischung wurde per GC ermittelt. In ausgewählten Beispielen wurde das Produkt bei 0,5 mbar und 75 bis 85°C durch fraktionierte Destillation isoliert.

### Beispiel 1

134 g (1 mol) Phthalid, 1,9 g (0,03 mol, 3 mol%) Borsäure und 8,5 g (0,03 mol, 3 mol%) Triphenylphosphinoxid wurden im Rührbehälter vorgelegt und auf 130°C erwärmt. Zu dieser Schmelze wurden innerhalb von 3 Stunden 155 g (1,3 mol) Thionylchlorid zugetropft. Anschließend wurde noch 5 Stunden bei 130°C nachgerührt. Der Reaktionsaustrag (183 g) enthielt 97 GC-Flächen-% o-Chlormethylbenzoylchlorid.

### Beispiel 2

670 g (5 mol) Phthalid, 26 g (0,25 mol, 5 mol%) Borsäuretrimethyl-ester und 70,5 g (0,25 mol, 5 mol%) Triphenylphosphinoxid wurden im Rührbehälter vorgelegt und auf 130°C erwärmt. Zu dieser Schmelze wurden innerhalb von 5 Stunden 774 g (6,5 mol) Thionylchlorid zugetropft. Anschließend wurde noch 5 Stunden bei 130°C nachgerührt. Nach Destillation des Reaktionsaustrags wurden 940 g (99,4 % Ausbeute) o-Chlormethylbenzoylchlorid in einer Reinheit von 98 % (GC) erhalten.

### Beispiel 3

268 g (2 mol) Phthalid, 10,4 g (0,1 mol, 5 mol%) Borsäuretrimethylester und 33,4 g (0,096 mol, 4,8 mol%) Cyanex® 923 sowie 310 g (2,6 mol) Thionylchlorid wurden im Rührbehälter vorgelegt und auf 120°C erwärmt. Anschließend wurde noch 4 Stunden bei 120°C nachgerührt. Der Reaktionsaustrag enthielt 84 GC-Flächen-% o-Chlormethylbenzoylchlorid und 9 % unumgesetztes Phthalid.

### Beispiel 4

268 g (2 mol) Phthalid, 14,8 g (0,1 mol, 5 mol%) Bortrifluorid-Etherat und 33,4 g (0,096 mol, 4,8 mol%) Cyanex® 923 sowie 310 g (2,6 mol) Thionylchlorid wurden im Rührbehälter vorgelegt und auf 100°C erwärmt. Anschließend wurde noch 15 Stunden bei 100°C nachgerührt. Der Reaktionsaustrag enthielt 93 GC-Flächen-% o-Chlormethylbenzoylchlorid und 2,8 % unumgesetztes Phthalid. Nach Destillation wurde das wertprodukt in einer Ausbeute von 89 % isoliert.

### Beispiel 5

268 g (2 mol) Phthalid, 17,8 g (0,12 mol, 6 mol%) Bortrifluorid-Etherat und 40 g (0,12 mol, 6 mol%) Triphenylphosphindichlorid sowie 310 g (2,6 mol) Thionylchlorid wurden im Rührbehälter vorgelegt und auf 100°C erwärmt. Anschließend wurde noch 15 Stunden bei 100°C nachgerührt. Der Reaktionsaustrag enthielt 92 GC-Flächen-% o-Chlormethylbenzoylchlorid und 5 % unumgesetztes Phthalid.

### Beispiel 6

134 g (1 mol) Phthalid, 7,9 g (0,05 mol, 5 mol%) Bortrifluorid-Dihydrat und 16,7 g (0,048 mol, 4,8 mol%) Cyanex® 923 sowie 155 g (1,3 mol) Thionylchlorid wurden im Rührbehälter vorgelegt und auf 100°C erwärmt. Anschließend wurde noch 7 Stunden bei 100°C nachgerührt. Der Reaktionsaustrag enthielt 83 GC-Flächen-% o-Chlormethylbenzoylchlorid und 7 % unumgesetztes Phthalid.

### Beispiel 7

268 g (2 mol) Phthalid, 10,4 g (0,1 mol, 5 mol%) Borsäuretrimethylester und 26,3 g (0,1 mol, 5 mol%) Triphenylphosphin wurden bei 130°C vorgelegt. Hierzu wurden über 5 Stunden 310 g (2,6 mol) Thionylchlorid zugetropft. Anschließend wurde noch 5 Stunden bei 130°C nachgerührt. Der Reaktionsaustrag enthielt 98 GC-Flächen-% o-Chlormethylbenzoylchlorid.

### Beispiel 8

Zu vorgelegten 13,4 g (0,1 mol) Phthalid, 1,08 g (5 mol%) Phenylboronsäuredi(isopropyl)ester und 1,4 g (5 mol%) Triphenylphosphinoxid wurden 15,5 g (0,13 mol) Thionylchlorid zugetropft. Anschließend wurde 10 Stunden bei 130°C gerührt. Der Reaktionsaustrag enthielt 86 GC-Flächen-% o-Chlormethylbenzoylchlorid.

### Beispiel 9

Eine Mischung von 13,4 g (0,1 mol) Phthalid, 15,5 g (0,13 mol) Thionylchlorid, 0,82 g (5 mol%) Trimethoxyboroxin und 1,4 g (5 mol%) Triphenylphosphinoxid wurden 10 Stunden bei 130°C gerührt. Der Reaktionsaustrag enthielt 95,4 GC-Flächen-% o-Chlormethylbenzoylchlorid.

## Patentansprüche

1. Verfahren zur Herstellung von o-Chlormethylbenzoesäurechloriden der Formel I, 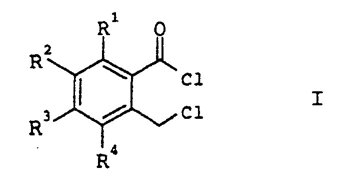 in der R¹ bis R⁴ gleich oder verschieden sein können und für Wasserstoff, C₁-C₄-Alkyl, Halogen oder Trifluormethyl stehen, durch Umsetzung von benzokondensierten Lactonen der Formel II, 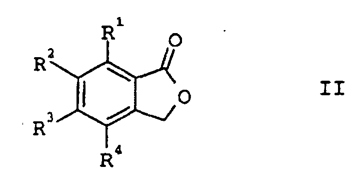 in der R¹ bis R⁴ die vorgenannte Bedeutung besitzen, mit Thionylchlorid,
**dadurch gekennzeichnet, daß** man die Umsetzung in Gegenwart katalytischer Mengen einer Lewis-Säure und katalytischer Mengen eines Phosphinderivats der Formel III 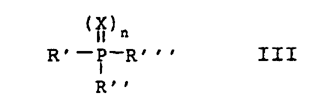 durchführt, in der R' bis R''' gleich oder verschieden sein können und C₁-C₁₀-Alkyl oder unsubstituiertes oder durch C₁-C₄-Alkyl substituiertes Phenyl bedeuten, der Index n für 0 oder 1 steht und X Sauerstoff oder zwei einfach gebundene Chloratome bedeutet.

2. Verfahren gemäß Anspruch 1, **dadurch gekennzeichnet, daß** als Lewis-Säure eine Borverbindung eingesetzt wird.

3. Verfahren gemäß Anspruch 1, **dadurch gekennzeichnet, daß** als Lewis-Säure Borsäure eingesetzt wird.

4. Verfahren gemäß Anspruch 1, **dadurch gekennzeichnet, daß** als Lewis-Säure Bortrifluorid oder Bortrichlorid in Komplex-gebundener Form eingesetzt wird.

5. Verfahren gemäß Anspruch 1, **dadurch gekennzeichnet, daß** als Lewis-Säure ein cyclischer Borsäureester oder ein Borsäuretri-C₁-C₄-alkylester eingesetzt wird.

6. Verfahren gemäß Anspruch 1, **dadurch gekennzeichnet, daß** als Lewis-Säure eine Boronsäure, ein Borsäureanhydrid oder ein Borat eingesetzt wird.

7. Verfahren gemäß einem der Ansprüche 1 bis 6, **dadurch gekennzeichnet, daß** die Lewis-Säure in einer Konzentration von 0,1 bis 20 mol-% bezogen auf das Lacton II eingesetzt wird.

8. Verfahren gemäß einem der Ansprüche 1 bis 7, **dadurch gekennzeichnet, daß** als Phosphinderivat Triphenylphosphinoxid eingesetzt wird.

9. Verfahren gemäß einem der Ansprüche 1 bis 6, **dadurch gekennzeichnet, daß** als Phosphinderivat ein bei Raumtemperatur flüssiges Trialkylphosphinoxid eingesetzt wird.

10. Verfahren gemäß einem der Ansprüche 1 bis 9, **dadurch gekennzeichnet, daß** 0,1 bis 20 mol-% des Phosphinderivats bezogen auf das Lacton II eingesetzt werden.

## Claims

1. A process for preparing o-chloromethylbenzoyl chlorides of the formula I 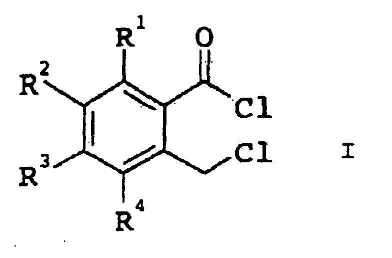 in which R¹ to R⁴ can be identical or different and are hydrogen, C₁-C₄-alkyl, halogen or trifluoromethyl, by reacting benzo-fused lactones of the formula II 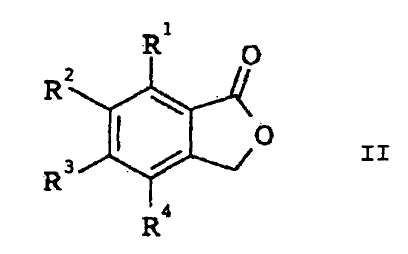 in which R¹ to R⁴ are as defined above with thionyl chloride, which comprises carrying out the reaction in the presence of catalytic amounts of a Lewis acid and catalytic amounts of a phosphine derivative of the formula III 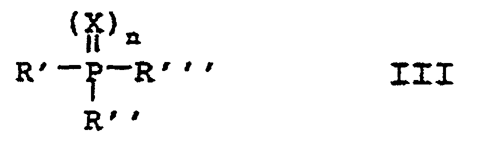 in which R' to R''' can be identical or different and are C₁-C₁₀-alkyl or unsubstituted or C₁-C₄-alkyl-substituted phenyl, the index n is 0 or 1 and X is oxygen or two singly attached chlorine atoms.

2. A process as claimed in claim 1, wherein the Lewis acid used is a boron compound.

3. A process as claimed in claim 1, wherein the Lewis acid used is boric acid.

4. A process as claimed in claim 1, wherein the Lewis acid used is boron trifluoride or boron trichloride in coordinate form.

5. A process as claimed in claim 1, wherein the Lewis acid used is a cyclic borate or a tri-C₁-C₄-alkyl borate.

6. A process as claimed in claim 1, wherein the Lewis acid used is a boronic acid, a boric anhydride or a borate.

7. A process as claimed in any of claims 1 to 6, wherein the Lewis acid is used in a concentration of from 0.1 to 20 mol%, based on the lactone II.

8. A process as claimed in any of claims 1 to 7, wherein the phosphine derivative used is triphenylphosphine oxide.

9. A process as claimed in any of claims 1 to 6, wherein the phosphine derivative used is a trialkylphosphine oxide which is liquid at room temperature.

10. A process as claimed in any of claims 1 to 9, wherein from 0.1 to 20 mol% of the phosphine derivative, based on the lactone II, are used.

## Revendications

1. Procédé de préparation de chlorures de l'acide o-chlorométhylbenzoïque de formule I, 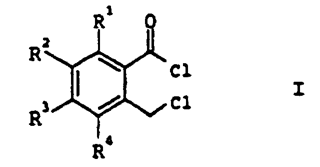 dans laquelle R¹ à R⁴ peuvent être identiques ou différents et représentent hydrogène, alkyle en C₁ à C₄, halogène ou trifluorométhyle, par transformation de lactones benzocondensées de formule II 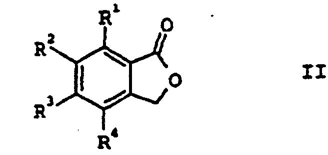 dans laquelle R¹ à R⁴ ont la signification susmentionnée, avec du chlorure de thionyle, **caractérisé en ce qu'**on réalise la transformation en présence de quantités catalytiques d'un acide de Lewis et de quantités catalytiques d'un dérivé de phosphine de formule III 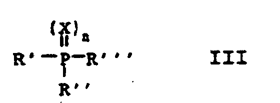 dans laquelle R' à R"' peuvent être identiques ou différents et signifient alkyle en C₁ à C₁₀ ou phényle non substitué ou substitué par alkyle en C₁ à C₄, l'indice n représente 0 ou 1 et X signifie oxygène ou deux atomes de chlore liés par une simple liaison.

2. Procédé selon la revendication 1, **caractérisé en ce qu'**on utilise un composé du bore comme acide de Lewis.

3. Procédé selon la revendication 1, **caractérisé en ce qu'**on utilise l'acide borique comme acide de Lewis.

4. Procédé selon la revendication 1, **caractérisé en ce qu'**on utilise du trifluorure de bore ou du trichlorure de bore sous une forme liée dans un complexe comme acide de Lewis.

5. Procédé selon la revendication 1, **caractérisé en ce qu'**on utilise un ester cyclique de l'acide borique ou un acide trialkylique en C₁ à C₄ de l'acide borique comme acide de Lewis.

6. Procédé selon la revendication 1, **caractérisé en ce qu'**on utilise un acide boronique, un anhydride de l'acide borique ou un borate comme acide de Lewis.

7. Procédé selon l'une quelconque des revendications 1 à 6, **caractérisé en ce que** l'acide de Lewis est utilisé en une concentration de 0,1 à 20% en mole par rapport à la lactone II.

8. Procédé selon l'une quelconque des revendications 1 à 7, **caractérisé en ce qu'**on utilise de l'oxyde de triphénylphosphine comme dérivé de phosphine.

9. Procédé selon l'une quelconque des revendications 1 à 6, **caractérisé en ce qu'**on utilise un oxyde de trialkylphosphine liquide à température ambiante comme dérivé de phosphine.

10. Procédé selon l'une quelconque des revendications 1 à 9, **caractérisé en ce qu'**on utilise 0,1 à 20% en mole du dérivé phosphine par rapport à la lactone II.
